# EUROPEAN PATENT SPECIFICATION

(11) **EP 2 911 642 B1**
(45) Date of publication and mention of the grant of the patent: **16.11.2022**
(21) Application number: 13848501.6
(22) Date of filing: 22.10.2013
(51) Int. Cl.: G16H 20/10

(54) **DRUG MONITORING AND REGULATION SYSTEMS AND METHODS**
MEDIKAMENTENÜBERWACHUNG UND REGELSYSTEME SOWIE VERFAHREN
SYSTÈMES ET PROCÉDÉS DE SURVEILLANCE ET DE RÉGULATION DE MÉDICAMENT

(30) Priority: 23.10.2012 US 201261717609 P
(43) Date of publication of application: 02.09.2015
(73) Proprietor: Labrador Diagnostics LLC, Wilmington, Delaware 19801 (US)
(72) Inventor: HOLMES, Elizabeth, Palo Alto, CA 94304 (US)
(74) Representative: Avidity IP
(86) International application number: PCT/US2013/066238
(87) International publication number: WO 2014/066428

(56) References cited:
- WO-A1-2008/111736
- WO-A1-2008/111736
- WO-A2-2007/064675
- US-A1- 2005 048 666
- US-A1- 2006 047 538
- US-A1- 2011 184 379
- US-B1- 7 124 031
- US-B2- 7 220 240
- US-B2- 7 220 240

## Description

### BACKGROUND

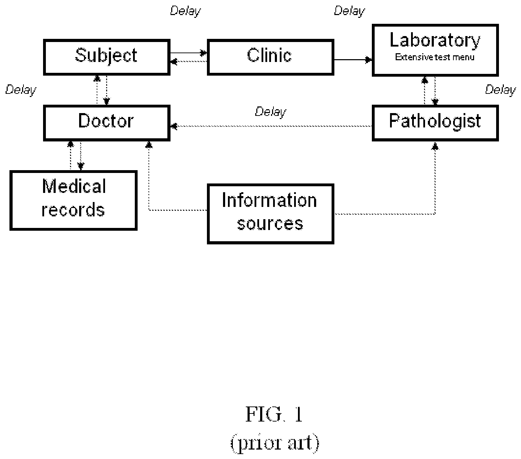

There are currently methods available in the art for monitoring and/or regulating the concentration of a drug in a patient. However, available methods typically are slow, complicated, error-prone, and/or imprecise. FIG. 1 schematically illustrates a process workflow for monitoring the concentration of a drug in a subject (e.g., patient). The arrows in the figure indicate the flow of information and/or samples to and from various entities illustrated in the figure. A doctor or technician may monitor the concentration of a drug in the subject. In some cases, the doctor may request that a clinic remove a sample of body tissue or fluid, such as blood or saliva, from the patient to monitor the concentration of the drug in the patient. In such a case, a technician, or other party authorized to withdraw a tissue sample from a patient, retrieves a blood or tissue sample from a patient and measures the concentration of a drug in the patient.

This typically requires the technician send the blood or tissue sample to a laboratory where such concentration is determined. In some situations, once the concentration of the drug has been determined, a specialist, such as a technician or pathologist, reviews the concentration to determine whether the dosage of drug for the patient needs to be increased, decreased (e.g., lowered, terminated), or not changed. The specialist then instructs the doctor by either communicating instructions to the doctor or inputting the instructions to an information source, such as a patient database. In the latter case, the doctor accesses information source to retrieve the pathologist's instructions. The doctor then instructs the patient to increase the dosage of the patient's drug, decrease the dosage, or keep the dosage constant. The doctor subsequently updates the patient's medical record in a medical records database. US 2011/0184379 discloses methods for treating hepatitis infections with interferon by a practitioner using pharmacokinetic and pharmacodynamic parameters obtained from data collected over time.

Improved systems and methods for monitoring the concentration of a drug in a patient and developing drug dosage regimens for patients are needed.

### SUMMARY

In accordance with the present invention there is provided a system for recommending a second dosage of a pharmaceutical composition as defined in the appended claims. Specific embodiments of the invention are defined in the dependent claims. The examples and embodiments not falling within the scope of the claims are for reference only.

In examples, methods for monitoring the concentration of a drug or a metabolite of the drug in a subject and of administering a drug to a subject are provided. In one example, a method for administering a pharmaceutical composition to a subject comprises administering to the subject a first dose of a pharmaceutical composition at a first time point; determining from a biological sample of said subject a concentration of an active ingredient of said pharmaceutical composition or a metabolite thereof, or a biological marker indicative of the concentration of said pharmaceutical composition in said subject, wherein said determining is carried out with the aid of a point of care system; and administering to said subject a second dose of said pharmaceutical composition at a second time point, wherein said second dose and/or the interval between said first and second time points is determined based on said concentration of said pharmaceutical composition in said subject.

In another example, a method for administering a pharmaceutical composition to a subject comprises administering to the subject a second dose of said pharmaceutical composition at a third time point, wherein said second dose and said third time point is determined by: (i) administering to said subject a first dose of a pharmaceutical composition at a first time point; (ii) determining from a biological sample of said subject a concentration of an active ingredient of said pharmaceutical composition or a metabolite thereof, or a biological marker indicative of the concentration of said pharmaceutical composition in said subject at a second time point, wherein said determining is carried out with a point of care system, thereby determining said second dose and the interval between said second and third time points based on said blood concentration determined at said second time point.

In another example, a method for monitoring the concentration of a pharmaceutical composition in a subject comprises prescribing to the subject a pharmaceutical composition at a first dose and at a first time; determining from a biological sample of said subject a concentration of an active ingredient of said pharmaceutical composition or a metabolite thereof, or a biological marker indicative of the concentration of said pharmaceutical composition in said subject with the aid of a point of care system at a predetermined interval; and prescribing to the subject a second dose of the pharmaceutical composition at a second time based on the determined blood concentration.

In other examples, systems for monitoring a concentration of a drug or a metabolite of the drug in a subject are provided. In an example, a system for monitoring a concentration of a drug in the subject comprises a point of care module for monitoring a concentration of an active ingredient of a pharmaceutical composition or a metabolite thereof, or a biological marker indicative of the concentration of said pharmaceutical composition in said subject; a drug monitoring module in communication with the point of care module, the drug monitoring module for providing one or more set point concentrations of an active ingredient of a pharmaceutical composition or a metabolite thereof in the subject; and a medical record module comprising records of doses in time, the medical record module for storing and providing a medical record of the subject.

In another example, a method for administering a pharmaceutical composition to a subject is provided, comprising: (a) administering to a subject a first dose of a pharmaceutical composition at a first time point; (b) obtaining a biological sample of said subject with the aid of a point of care system; (c) measuring, with the aid of the point of care system and the use of said biological sample, concentration of an active ingredient of said pharmaceutical composition or a metabolite thereof, or a biological marker indicative of the concentration of said pharmaceutical composition present in said subject; (d) calculating, with the aid of a computer processor, dosage and/or time interval between dosages based on said concentration measured in step (c); and (e) administering to said subject a second dose of said pharmaceutical composition at a second time point according to said calculated dosage and/or time interval of step (d). Step (a) may be carried out with the aid of the point of care system and without the involvement of any human other than the subject. Step (c) may be carried out with the aid of the point of care system and without the involvement of any human other than the subject. Step (e) may be carried out with the aid of the point of care system and without the involvement of any human other than the subject. Step (c) may comprise measuring the blood concentration of said active ingredient of said pharmaceutical composition or a metabolite thereof. The method may further comprise comparing said concentration to one or more set-point concentrations; and calculating the second dose based on the difference between said concentration and the one or more set-point concentrations. The method may further comprise monitoring, with the aid of a computer processor, the progression of a health condition of said subject in response to administering said first dose and/or said second dose. The method may further comprise adjusting said first dose, said second dose and/or said interval based on said monitoring.

In another example, a method for administering a pharmaceutical composition to a subject is provided, comprising: administering to a subject a second dose of said pharmaceutical composition at a third time point, wherein said second dose and said third time point is determined by: (a) administering to said subject a first dose of a pharmaceutical composition at a first time point; (b) obtaining a biological sample of said subject with the aid of a point of care system; (c) measuring, with the aid of the point of care system and the user of said biological sample, concentration of an active ingredient of said pharmaceutical composition or a metabolite thereof, or a biological marker indicative of the concentration of said pharmaceutical composition present in said subject at a second time point; and (d) calculating, with the aid of a computer processor, dosage and/or time interval between dosages based on said concentration measured in step (c), thereby determining said second dose and/or the interval between said second and third time points. Step (c) may comprise measuring the blood concentration of said active ingredient of said pharmaceutical composition or a metabolite thereof. The method may further comprise comparing said concentration to one or more set-point concentrations; and calculating the second dose based on the difference between said concentration and the one or more set-point concentrations. The method may further comprise monitoring, with the aid of a computer processor, the progression of a health condition of said subject in response to administering said first dose and/or said second dose. The method may further comprise adjusting said first dose, said second dose and/or said interval based on said monitoring.

In another example, a method for monitoring the concentration of a pharmaceutical composition in a subject is provided, comprising: (a) prescribing to a subject a first dose of a pharmaceutical composition at a first time; (b) obtaining a biological sample of said subject with the aid of a point of care system; (c) measuring, with the aid of the point of care system and the user of the said biological sample, concentration of an active ingredient of said pharmaceutical composition or a metabolite thereof, or a biological marker indicative of the concentration of said pharmaceutical composition present in said subject; and (d) calculating, with the aid of a computer processor, dosage and/or time interval between dosages based on said concentration measured in step (c); and (e) prescribing to the subject a second dose of the pharmaceutical composition at a second time according to said calculated dosage and/or time interval of step (d). The method may further comprise comparing, with the aid of a processor, said measured concentration of step (c) to the one or more set-point concentrations; and calculating the second dose based on the difference between said measured concentration and the one or more set-point concentrations. The method may further comprise monitoring, with the aid of a computer processor, the progression of a health condition of said subject in response to prescribing said first dose and/or said second dose. The method may further comprise determining said first dose, said second dose and/or said predetermined interval based on said monitoring.

In another example, a system for monitoring a concentration of a drug in a subject is provided, comprising: a point of care module for monitoring, with the aid of a computer processor, a concentration of an active ingredient of a pharmaceutical composition or a metabolite thereof, or a biological marker indicative of the concentration of said pharmaceutical composition in said subject: a drug monitoring module in communication with the point of care module, the drug monitoring module for providing one or more set point concentrations of an active ingredient of a pharmaceutical composition or a metabolite thereof in the subject; and a medical record module comprising records of doses in time, the medical record module for storing and providing a medical record of the subject. In some examples, the drug monitoring module may provide the one or more set point concentrations with the aid of a predetermined table. In some examples, the drug monitoring module may provide the one or more set point concentrations with the aid of a learning engine. In some examples, the system may further comprise an output module for providing one or more instructions to the subject. The one or more instructions may be selected from dose instructions and administration instructions for a pharmaceutical composition. In some examples, the system may further comprise a server in communication with the point of care module and the medical records module. The server may include one or more of a learning engine, protocols and calibration protocols.

In another example, a method for recommending a dose of a pharmaceutical composition to a subject is provided, comprising: administering to the subject at a first point in time a dose of the pharmaceutical composition; using a point of care system to determine concentration data for an active ingredient of said pharmaceutical composition, a metabolite of an active ingredient of said pharmaceutical composition, or a biological marker indicative of the concentration of said pharmaceutical composition in said subject; comparing, by a computing device, a) the concentration data and b) at least one set point value, wherein the set point value is stored in computer memory, retrieved from a data structure, or calculated by the computing device; in response to comparing the concentration data and the at least one set point value, directing the computing device to determine if at least one change in a dosing regimen for the pharmaceutical composition is desired in order to have the concentration of said pharmaceutical composition be in a desired range, said computing device utilizing a predictive model based at least in part on pharmacokinetic data of the pharmaceutical composition; and generating a recommendation for at least a next dose of said pharmaceutical composition, wherein the recommendation sets forth at least one of: a) an amount of the next dose or b) a time for applying the next dose. In some examples, the method may further comprise directing a processor to modify a patient history data structure to store concentration data related to the pharmaceutical composition administered at the first point in time, wherein the patient history data structure is configured to store longitudinal data regarding concentration from other points in time.

In some examples, in a system, method, or device described above or elsewhere herein involving administering to a subject a first dose of a pharmaceutical composition, the administering is carried out with the aid of the point of care system and without the involvement of any human other than the subject.

In some examples, in a system, method, or device described above or elsewhere herein involving measuring, with the aid of the point of care system and the use of said biological sample, concentration of an active ingredient of a pharmaceutical composition or a metabolite thereof, or a biological marker indicative of the concentration of a pharmaceutical composition present in a subject, the measuring is carried out with the aid of the point of care system and without the involvement of any human other than the subject.

In some examples, in a system, method, or device described above or elsewhere herein involving administering to a subject a second dose of a pharmaceutical composition, the administering is carried out with the aid of the point of care system and without the involvement of any human other than the subject.

In some examples, in a system, method, or device described above or elsewhere herein involving comparing a concentration to one or more set-point concentrations, the comparing comprises comparing the concentration to a subject-specific maximum drug concentration and a subject-specific minimum drug concentration.

In some examples, in a system, method, or device described above or elsewhere herein involving subject-specific maximum and subject-specific minimum drug concentrations, the subject-specific maximum and subject-specific minimum drug concentrations are determined based on maximum concentrations and minimum concentrations of a plurality of subject-specific drug concentration profiles.

In some examples, in a system, method, or device described above or elsewhere herein involving comparing a drug concentration to one or more set-point concentrations, the comparing comprises comparing the drug concentration to an average set point concentration.

In some examples, in a system, method, or device described above or elsewhere herein involving an average set-point concentration, the average set-point concentration is a time-averaged set-point concentration.

In some examples, in a system, method, or device described above or elsewhere herein involving one or more set-point concentrations, the one or more set-point concentrations are subject-specific set-point concentrations.

In some examples, in a system, method, or device described above or elsewhere herein involving measuring the blood concentration of the active ingredient of a pharmaceutical composition or a metabolite thereof, the blood concentration is measured in real-time.

In some examples, in a system, method, or device described above or elsewhere herein involving measuring the blood concentration of the active ingredient of a pharmaceutical composition or a metabolite thereof, the blood concentration is measured at predetermined intervals.

In some examples, in a system, method, or device described above or elsewhere herein involving a point of care system, the point of care system is operatively coupled to the blood or tissue of the subject.

In some examples, in a system, method, or device described above or elsewhere herein involving a biological sample, the biological sample is blood.

This Summary is provided to introduce a selection of concepts in a simplified form that are further described below in the Detailed Description. Additional aspects and advantages of the present disclosure will become readily apparent to those skilled in this art from the following Detailed Description. As will be realized, the present disclosure is capable of other and different examples, and its several details are capable of modifications in various obvious respects, all without departing from the disclosure. Accordingly, the drawings and description are to be regarded as illustrative in nature, and not as restrictive.

### BRIEF DESCRIPTION OF THE DRAWINGS

In the drawings,
FIG. 1 schematically illustrates a conventional process workflow for monitoring the concentration of a drug in a subject.
FIG. 2 schematically illustrates a process workflow provided herein for monitoring the concentration of a drug (or metabolite of the drug) in a subject.
FIG. 3 schematically illustrates a system provided herein for monitoring the concentration of a drug (or metabolite of the drug) in a subject.
FIG. 4 schematically illustrates a method provided herein for monitoring the concentration of a drug (or metabolite of the drug) in a subject.
FIGs. 5-11 show blood concentration profiles of a subject, in accordance with various systems and methods provided herein.

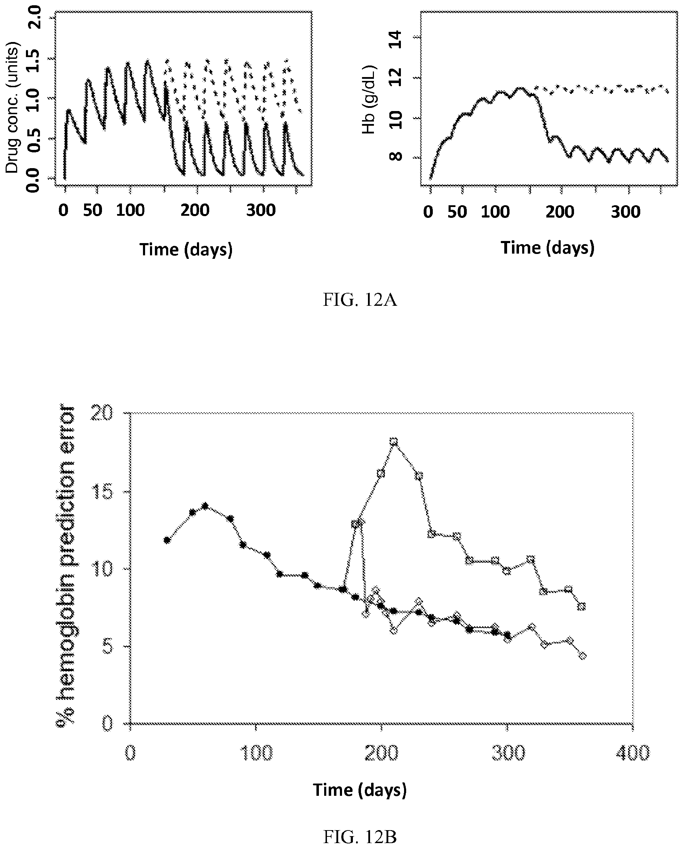

FIG. 12A shows "real" patient profiles for serum drug concentration and hemoglobin. The solid line shows the profiles when a change in behavior occurred at time 150 days. The dotted line indicates the profiles in the absence of the change in behavior. FIG. 12B shows prediction errors associated with the patient profiles of FIG. 12A. The profile with the solid circles indicates the prediction error when no change in patient behavior occurred, and the profiles with the open squares (unchanged sampling frequency) and the open diamonds (increased sampling frequency) show the prediction errors when a behavior change occurred at time = 150 days. The unexpected error increase for these two cases after 150 days indicates the occurrence of a behavior change.

### DETAILED DESCRIPTION

While various embodiments are shown and described herein, it will be obvious to those skilled in the art that such embodiments are provided by way of example only. Numerous variations, changes, and substitutions may occur to those skilled in the art without departing from the disclosures provided herein.

The term "point of service system," as used herein, refers to a system that is capable of providing a service (e.g. testing, monitoring, treatment, diagnosis, guidance, sample collection, ID verification, medical services, non-medical services, etc.) at or near the site or location of the subject. In some situations, a point of service system provides a service at a predetermined location, such as a subject's home or work, grocery stores, drug stores, clinics, schools, etc. A point of service system may include a computer processor. In some cases, a point of service system can include a remote system for data analysis and/or storage. Such a remote system can include a computer processor and memory with machine executable code for implementing various operations, such as data analysis. A point of service system can include one or more point of service devices. In some examples, a point of service system is a point of care system. A "point of care system" refers to a system that is capable of providing medical-related care (e.g. treatment, testing, monitoring, diagnosis, counseling, etc.) at or near the site or location of the subject (e.g. at a subject's home or work, grocery stores, drug stores, clinics schools, etc.).

The term "subject," as used herein, refers to an individual that in the past was, currently is, or in the future may be under the care of, or acted upon by, a point of service system. A subject can be an individual whose health (e.g., physical condition), or chemical and/or biological state, is being monitored or that is in need of treatment or monitoring. A subject can include a patient. In some cases, a subject is a human patient. A subject can be a user of a point of service device (or system), or computer system associated with the point of service device. In some cases the terms "subject" and "user" are used interchangeably.

The term "regimen," as user herein, refers to a systematic plan that may improve and maintain the health of a subject.

The term "dose," as used herein, refers to a quantity of a drug prescribed, administered or taken at a particular time or over an interval. In some situations, a dose may include an optimum therapeutic dose and optimum interval between doses.

The term "drug," as used herein, refers to a pharmaceutical composition, such as a chemical or biological substance for treating a subject. A drug includes a pharmacologically active ingredient. In some situations, a drug may include a pharmacologically inactive ingredient, such as an excipient. In some situations, the concentration or amount of a drug / pharmaceutical composition may be determined by measuring the level of the pharmaceutical composition itself, the level of a metabolite of the pharmaceutical composition, or the level of a biological marker indicative of the concentration of the pharmaceutical composition in the subject. The term "pharmacokinetics," as used herein, refers to the study of the mechanisms of absorption and distribution of an administered drug, the rate at which a drug action begins and the duration of the effect, the chemical changes of the substance in the body (e.g., by enzymes) and the effects and routes of excretion of the metabolites of the drug.

The term "pharmacodynamics," as used herein, refers to the study of the biochemical and physiological effects of drugs on the body of a subject or on microorganisms or parasites within or on the body and the mechanisms of drug action and the relationship between drug concentration and effect.

The term "communicatively coupled," as used herein, refers to direct or indirect communication between entities, such as devices, a user and a device, or users. For instance, a user is communicatively coupled to a device when the user is in communication with another user or device either directly (i.e., direction communication) or indirectly (i.e., indirect communication).

The term "biological marker," as used herein, refers to one or more species or substances that can be indicative of the concentration of a pharmaceutical composition in a subject. A biological marker may include, without limitation, drugs, prodrugs, pharmaceutical agents, drug metabolites, biomarkers such as expressed proteins and cell markers, antibodies, serum proteins, cholesterol, polysaccharides, nucleic acids, biological analytes, biomarker, gene, protein, lipids or hormone, or any combination thereof. Biomarkers can be associated with, for example, autoimmune diseases, obesity, hypertension, diabetes, neuronal and/or muscular degenerative diseases, cardiac diseases, endocrine disorders, cancers, infections, and any combinations thereof. Biomarkers can be present in varying abundance in one or more of body tissues, including heart, liver, prostate, lung, kidney, bone marrow, blood, skin, bladder, brain, muscles, nerves, and selected tissues that are affected by various disease, such as different types of cancer (malignant or non-metastatic), autoimmune diseases, inflammatory and/or degenerative diseases. Other examples of biological markers are provided in U.S. Patent Application No. 12/412,334 to Michelson et al. ("METHODS AND SYSTEMS FOR ASSESSING CLINICAL OUTCOMES").

The term "physical indicator," as used herein, refers to a physically recognizable or measurable characteristic of a subject that can be indicative of a health of a subject and/or the subject's response to therapy (e.g., drug treatment), such as, for example, heart rate, pupil dilation, breathing rate, perspiration, body temperature, urine concentration, dizziness, and facial expression.

Recognized herein are various limitations associated with current methods for monitoring and regulating the concentration of a drug or a metabolite of the drug in a subject. For example, the flow of information and acquisition of tissue samples from a patient, subsequent testing in a laboratory, and review by a doctor and/or pathologist, such as according to the workflow of FIG. 1, may be a time intensive process, requiring in some cases several days or even weeks to complete. The time scale from acquiring a tissue sample to reporting the concentration of a drug to a doctor may be on the order of days, weeks or even months. This is problematic when the time scale of a patient's response to drug regimen is on the order of seconds, minutes, hours, or days, which may require monitoring and adjusting the concentration of the drug (or metabolite of the drug) on a time scale that is on the order of seconds or minutes. Based on the approach of FIG. 1, when the concentration of a drug or a metabolite of the drug in the body of a subject has been determined, that concentration may not be reflective of the then concentration of the drug or metabolite of the drug in the body of the subject. Any subsequent adjustment of the dosage of the drug may be inaccurate, leading a doctor to underestimate or overestimate a new dosage in an effort to effect a desirable blood concentration. In addition, because the approach of FIG. 1 includes numerous parties, the approach may be costly to a subject (e.g., patient).

Provided herein are point of care systems and methods for reducing, if not eliminating, the limitations associated with the method of FIG. 1. The disclosure provides point of care systems for enabling a subject to monitor the concentration of a drug (or metabolite of the drug) in the subject's body and modify a dosage regimen of the drug to maintain a desirable or predetermined concentration of the drug in the subject's body. In some examples, the point of care system automatically monitors the concentration of the drug (or metabolite of the drug) and regulates (e.g., adjusts) the dose of the drug to maintain a desirable concentration of the drug in the subject.

Point of care systems provided herein can regulate the concentration of a drug or metabolite of the drug in the body of a subject, and maintain the concentration to within a fluctuation of no more than about 50%, 40%, 30%, 25%, 20%, 15%, 10%, 5%, 4%, 3%, 2%, 1%, 0.5%, 0.1%, 0.01%, 0.001%, or 0.0001% greater or less than a target concentration. In some examples, a point of care system can measure the concentration of a drug or metabolite thereof in the subject (e.g., in the blood of the subject) at a rate of one measurement in a time period of no more than 1 week, 72 hours, 48 hours, 24 hours, 12 hours, 8 hours, 4 hours, 2 hours, 60 minutes, 30 minutes, 25 minutes, 20 minutes, 15 minutes, 10 minutes, 5 minutes, 4 minutes, 3 minutes, 2 minutes, 1 minutes, 30 seconds, 10 seconds, 5 seconds, 1 second, 0.5 seconds, or 0.1 seconds. In some examples, a point of care system can measure the concentration of a drug or metabolite thereof in a subject (e.g., in the blood of the subject), compare the concentration to one or more set-point concentrations, and adjust the concentration of the drug in view of the comparison, all within a time period of no more than about 1 week, 72 hours, 48 hours, 24 hours, 12 hours, 8 hours, 4 hours, 2 hours, 60 minutes, 30 minutes, 25 minutes, 20 minutes, 15 minutes, 10 minutes, 5 minutes, 4 minutes, 3 minutes, 2 minutes, 1 minutes, 30 seconds, 10 seconds, 5 seconds, or even 1 second.

In some examples, a point of care system can measure the concentration of one or more biomarkers (e.g., a plurality of different biomarkers) in a subject (e.g., in the blood of the subject) at a rate of one measurement in a time period of no more than 1 week, 72 hours, 48 hours, 24 hours, 12 hours, 8 hours, 4 hours, 2 hours, 60 minutes, 30 minutes, 25 minutes, 20 minutes, 15 minutes, 10 minutes, 5 minutes, 4 minutes, 3 minutes, 2 minutes, 1 minutes, 30 seconds, 10 seconds, 5 seconds, 1 second, 0.5 seconds, or 0.1 seconds. In some examples, a point of care system can measure the concentration of one or more biomarkers (e.g., a plurality of different biomarkers) in a subject (e.g., in the blood of the subject), compare the concentration to one or more set-point concentrations, and adjust the concentration of a drug in view of the comparison, all within a time period of no more than about 1 week, 72 hours, 48 hours, 24 hours, 12 hours, 8 hours, 4 hours, 2 hours, 60 minutes, 30 minutes, 25 minutes, 20 minutes, 15 minutes, 10 minutes, 5 minutes, 4 minutes, 3 minutes, 2 minutes, 1 minutes, 30 seconds, 10 seconds, 5 seconds, or even 1 second.

In some cases, the point of care system can automatically regulate the concentration of the drug in the subject by adjusting the dose of a drug supply to the subject. For example, if a drug is administered to the subject with the aid of a fluid flow path regulated by a controller, the point of care system can instruct the controller to adjust the dose. In other cases, the point of care system can instruct the subject to adjust the subject's dose manually. For example, the point of care system can instruct the subject to change the subject's dose from once a day to twice a day. The point of care system can provide such instructions to the subject with the aid of a graphical user interface (GUI) on an electronic display, which may be an electronic display on an electronic device of the subject (e.g., Smart phone, portable computer).

In some examples, methods and systems for the automated measurement and optimization of a drug dosage regimen are described. In an implementation, the integration of communications enabled by a mobile communication device allows for rapid responses to changing medical situations. In some cases, this requires changing an analytical parameter to be measured, the schedule for monitoring of one or more parameters, or an immediate change in a subject's therapy.

Systems and methods are provided for monitoring a drug regimen or a subject's response to a therapy and for making adjustments to the subject's therapy in response to the measurements. In some situations, the methods include using a point of care ("POC") system to (1) collect a fluid or tissue sample from the subject, (2) perform one or more tests or measurements on the fluid or tissue sample (e.g., with the aid of an assay, such as a bioassay), (3) interpret the data, which includes calculating the concentration of a drug or metabolite of the drug in the subject, (4) distribute information used to monitor the response of the subject to therapy, and (5) acquire pertinent clinical signs and symptoms as a function of a subject's biological state (e.g., as represented by a biomarker signature). In some examples, the methods are performed without any intervention from a healthcare provider. That is, the POC system may perform all of the aforementioned steps. However, in some situations, the POC system may alert a healthcare provider (e.g., doctor) if the POC system detects an abnormality, such as an irregular response to a therapy.

### Drug monitoring systems

In some examples, systems for monitoring the concentration of a drug or a metabolite of a drug in a subject are provided. In some examples, such systems are point of care ("POC") systems configured to provide monitoring at predetermined intervals or in real-time. In some situations, the intervals are calculated or regulated, or set by a user. Users include a subject under treatment or a healthcare provider (e.g., doctor, nurse, technician). In other situations, the intervals are calculated or otherwise determined by the system, such as with the aid of the POC system that determines when a measurement is required.

In some examples, a system for monitoring a concentration of a drug in a subject, includes a point of care module for monitoring a concentration of an active and/or inactive ingredient of a pharmaceutical composition (or a metabolite of the pharmaceutical composition), or a biological marker indicative of the concentration of the pharmaceutical composition in the subject. A drug-monitoring module is in communication with the point of care module. The drug-monitoring module is for providing one or more set-point concentrations of an active and/or inactive ingredient of the pharmaceutical composition (or a metabolite thereof) in the subject. A medical record module is in communication with the POC module. The medical record module comprises records of doses in time; the medical record module is for storing and providing a medical record of the subject to the POC module.

One or more modules may be provided in separate systems or included in the same system. For instance, the POC system may include the POC module, the drug monitoring module and the medical record module. As another example, the POC system may include the POC module and the drug-monitoring module; the medical record module in such a case is provided in a separate system in communication with the POC system. For example, the medical record module may be provided in a medical records system in network communication with the POC system.

The drug-monitoring module may provide the one or more set point concentrations with the aid of a predetermined table, such as a table of doses, which may be formed or updated by the system. The table may be formed specifically for the subject under treatment, or formed with the aid of concentration profiles across a predetermined population. In some implementations, the drug-monitoring module provides the one or more set point concentrations with the aid of a learning engine. The learning engine is configured to learn from and adapt to a subject's physiology, such as the subject's metabolism, which may enable the POC system to provide subject-specific monitoring and regulation of the concentration of a drug in the subject.

In some cases, a learning engine is provided with desired outcomes of therapy. Starting from a default initial dose regime, as data accumulates for particular individual patients and/or groups of patients and the dose regime is changed (initially according to a default algorithm), the learning engine associates regimes which work best for the subject(s) in order to design new regimes which may be better. The learning engine learns these patterns or associations by a collection of methods, including, without limitation, inference, statistical modeling, time series analysis, survival analysis, clustering, mixed effects modeling, mechanistic modeling, parameter estimation, uncertainty analysis, optimization, and/or machine learning methods.

The POC system may include an output module for providing one or more instructions to the subject. The one or more instructions may be selected from dose instructions and administration instructions for a pharmaceutical composition (or drug). For instance, the output module may instruct the subject to administer a dose of the drug at a certain time and at a particular dose (e.g., "Take 20 mg of penicillin at 10 AM").

In some situations, the POC system further includes a server in communication with the point of care module and the medical records module. The server includes one or more of a learning engine, protocols and calibration protocols, which may be used to calculate a dose of a drug for administration to the subject.

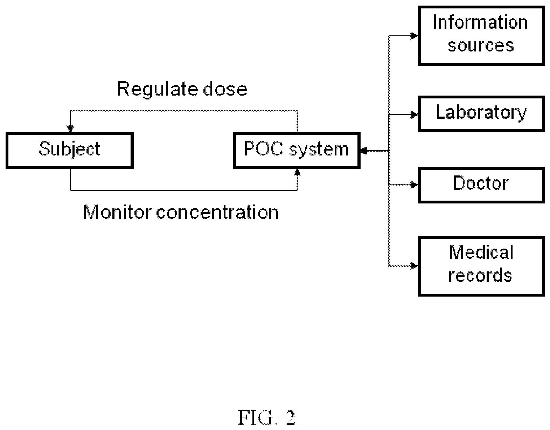

FIG. 2 illustrates a point of care workflow for monitoring the concentration of a drug in a subject, in accordance with a method and system provided herein. The point of care (POC) system monitors the concentration of the drug at predetermined intervals, such as on the order of microseconds, milliseconds, seconds, minutes, hours or days, or in intervals calculated to be optimal based on the collected data. In some situations, the POC system actively monitors the concentration of the drug in the subject. In other situations, the POC monitors the concentration of the drug in the subject upon the subject or another user (e.g., healthcare provider) requesting such monitoring.

In some examples, the POC system actively monitors the concentration of a drug, a metabolite of the drug or a marker indicative of the concentration of the drug without any involvement from a healthcare provider (e.g., physician). That is, the POC system may establish a direct feedback loop with the subject without any involvement with a healthcare provider. In some cases, the POC system monitors and regulates the concentration of the drug without the involvement of any human other than the subject. In such a case, the subject may be involved only to the extent that the subject is being acted upon by the POC system. In some situations, however, the POC system contacts a healthcare provider if the POC system detects an abnormality, such as an irregular response to treatment.

The POC system of FIG. 2 is in communication with one or more information services, such as information sources, a laboratory, a doctor (or other care provider), and a medical records database. Information sources inform the POC system of an acceptable blood concentration of a particular drug in a subject. This may be determined via clinical trials, or by the POC system with the aid of a learning engine. Such acceptable blood concentration may be an average acceptable blood concentration that is a function of the subject's age, sex, or other physiological traits, such as genetic factors (e.g., the presence of a particular gene predisposes the subject to metabolize a particular drug at a particular rate or to respond to a particular concentration of a drug in a certain way). The POC system may be in communication with a laboratory for retrieving test results, such as the concentration of a drug in a tissue from a biopsy. The POC system may be communicatively coupled to a doctor (or other healthcare provider), which enables the POC system to retrieve the subject's records, for example, or retrieve historical data as to the administration of a particular drug to the subject.

The POC system may access the subject's medical history with the aid of a medical records database. The medical records database may include one more computer systems with memory and other physical storage devices or systems. The medical records database may include a data warehouse, in addition to a file repository for storing one or more records of the subject. The data warehouse may include analysis and trending capabilities for analyzing and forecasting concentration profiles.

With continued reference to FIG. 2, the POC system may include one or more computers for computing and processing concentration data from a fluid or tissue sample from the subject. Each computer can have one or more processors, and a physical storage medium (e.g., flash memory, hard disk). For example, the POC system may include a single processor or multiprocessor system, the latter system for parallel processing. In addition, the POC system may include a tissue collection module for collecting a blood, fluid or tissue sample from the patient for measuring the concentration of a drug in the subject.

In some cases, the POC system is in communication with one or more probes for collecting concentration data in real-time or at intervals, such as a predetermined interval. For instance, a probe may measure the concentration of a drug in the blood of a subject at least every 1 minute, or 10 minutes, or 30 minutes, or 1 hour, or 2 hours, or 3 hours, or 4 hours, or 5 hours, or 6 hours, or 7 hours, or 8 hours, or 9 hours, or 10 hours, or 11 hours, or 12 hours, or 1 day, or at non-regular intervals. The one or more probes may be in fluid communication with a subject's blood. For instance, the one or more probes may be configured to circulate through the subject's body and transmit data to the POC system. In some cases, the one or more probes transmit data to an electronic relay (e.g., hub or transmitter) that is configured to transmit data to the POC system. The electronic relay is in proximity to the subject. For example, the electronic relay may be configured to be attached to the subject's wrist or arm.

In some examples, the POC system of FIG. 2 is configured to monitor the blood concentration of a particular drug by collecting blood concentration data from a subject being monitored with the aid of an assay. The assay may be performed by a health care provider, such as a doctor or laboratory technician. In some cases, the assay is performed with the aid of a machine or device for collecting a fluid (e.g., blood, saliva) or tissue sample from the subject.

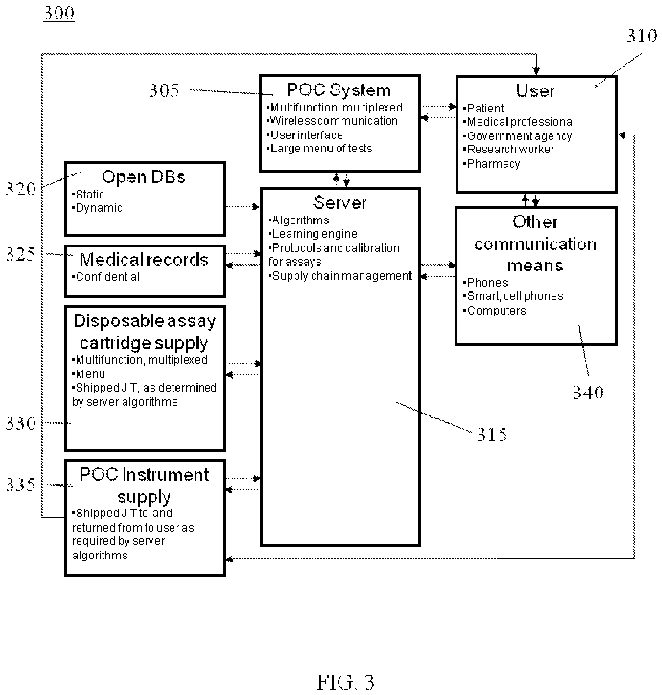

FIG. 3 schematically illustrates a system 300 for collecting, monitoring and regulating the concentration of a drug or metabolite of the drug in a subject provided herein. The system of FIG. 3 includes a point of care ("POC") system 305 in communication with a user 310, a server 315, a database (DB) 320, a medical records system 325, a disposable assay cartridge supply 330, and a POC instrument supply 335.

The user 310 may include one or more of a subject (e.g., patient), a medical or healthcare professional, a government agency, a research worker and a pharmacy. In some instances the user 310 is a patient under treatment. In such a case, the POC system 305 may be in direct communication with the user 310 to monitor the concentration of a drug or metabolite of the drug in the user 310 and make any necessary changes to the dose of the drug. The POC system 305 may then instruct the user 310 to make necessary changes to the dose of the drug (e.g., increase the dose, decrease the dose, or terminate the dose), or do nothing, which may be a desirable course of action if the measured concentration of the drug or metabolite of the drug is determined by the POC system 305 to be acceptable (e.g., within the targeted concentration range for the subject).

With continued reference to FIG. 3, the POC system 305 is in communication with the server 315 with the aid of a communication system, such as, for example, a wired or wireless network, Bluetooth, or other mode of communication, such as Universal Serial Bus (USB). The server 315 includes algorithms and rules for modifying a drug dose in response to a concentration of a drug or a metabolite of the drug in a subject. The server 315 also includes a learning engine for adapting a dosage regimen to a subject upon collecting one or a plurality of concentration profiles, or other physiological parameters (e.g., height, weight, sex), from the subject. The learning engine may include one or more trending algorithms for forecasting a subject's response to a change in the dose of a drug for the subject. The server 315 includes various protocols and calibration parameters that may be used for assays and other approaches for measuring the concentration of a drug or a metabolite of the drug in a subject. The learning engine is also configured to use all available information to construct better therapeutic regimes according to a heuristic model. The learning engine can be configured to balance the risks associated with potential adverse events associated with the drug against the potential benefits induced by the drug. The learning engine can be configured to assess uncertainty in model predictions and to use this information to better make drug dosing changes or recommendations, such as for the patient, healthcare provider or pharmacy. With the collection of more data, the learning engine progressively becomes more predictive and less uncertain. Changes, possibly sudden, in the physiology of the patient, characteristics of the drug, or other changes impacting the subject's response to the drug, can be detected by the learning engine, thereby inducing the learning engine to adjust or update its models and recommendations for the subject and also to collect additional data regarding the drug and the subject's response.

The server 315 is in communication with the database 320, which may store information for use by the learning engine of the server 315 for aiding the POC system 305 to monitor the concentration of a drug or metabolite of the drug in a subject. The database 320 may store additional information, such as algorithms or rules for use by the server 315. In some situations, the database is in communication with a data warehouse for trending and forecasting concentration profiles or other pertinent metrics associated with a subject's treatment. In some examples, the database 320 may include a static database component comprising static data related to drug being monitored and/or the population, and a dynamic database component comprising dynamic data about the population and individual subjects. In addition, the database 320 (or data warehouse in communication with the database 320) may include a computer-modeling component that is configured to model the data in the static database component and the dynamic database component, which enable modeling drug profiles (including concentrations profiles) within the population.

In some examples, the database 320 includes features described in U.S. Patent Application No. 12/906,975 ("INTEGRATED HEALTH DATA CAPTURE AND ANALYSIS SYSTEM").

The medical records system 325 may include one or more public or confidential information with respect to a subject under treatment. In some instances, the medical records system 325 includes the subject's insurance information, such as if the subject is qualified under the subject's insurance policy to be monitored by the system 300. The medical records system 325 also includes the subject's medical history, which may include dose history for a particular drug, whether the subject has used the drug, and whether the subject has had any adverse reactions to the particular drug.

The disposable assay cartridge supply 330 is configured to direct one or more assays to the user 310, as required, and upon instruction from the server 315. Assays may be directed to the user 310 by one or more shipping options, such as, for example, just-in-time (JIT) shipping. For instance, when the POC system 305 determines that measurement of the concentration of a drug or a metabolite of the drug in a subject is required, the server 315 instructs the disposable assay cartridge system 330 to direct one or more assays, such as disposable assay cartridges, to the user 310, which may be the subject under treatment. The user 310 may subsequently use the assay cartridge to collect and measure the concentration of a drug or a metabolite of the drug in the user. Alternatively, the disposable assay cartridge supply may direct one or more assay disposables to the user 310 on a predetermined interval, such as on a daily, weekly, monthly, or yearly basis, and the user 310 may use an assay, as required, such as upon instruction from the POC system 305 (with the aid of algorithms from the server 315, for example) to use the assay to measure the concentration of the drug (or a metabolite of the drug) in a subject under treatment. Alternatively, when the POC system 305 determines that measurement of the concentration of a drug or a metabolite of the drug in a subject is required, the system can execute only the required assays that are pre-loaded onto the disposable assay cartridges.

The POC instrument supply 335 is configured to provide a POC instrument to the user 310 upon instruction from the POC system 305. In some situations, the POC system 305 instructs the POC instrument supply to direct a POC instrument to the user 310 upon the server 305 instructing the POC system that a POC instrument is required. The POC instrument supply 335 directs a POC instrument to the user 310 by one or more shipping options, such as, for example, JIT shipping.

A POC instrument may include one or more devices for collecting a fluid or tissue sample from a subject with the aid of the user 310. In some situations the user 310 is the subject under treatment or monitoring. The POC instrument may subsequently measure the concentration of a drug or a metabolite of the drug in the subject. The measured concentration is subsequently directed to the POC system 305, which, with the aid of the server 315, will determine whether the concentration of the drug (or metabolite of the drug) is acceptable, or whether the concentration of the drug (or metabolite of the drug) is within or outside one or more limits or thresholds (see below). The POC system 305 may subsequently do nothing, or take remedial actions intended to bring the concentration of the drug within predetermined or desirable limits.

The server 315 may communicate with the user 310 with the aid of one or more communications devices 340, such as a telephone ("phone"), Smart phone (e.g., Apple^{®} iPhone^{®}), cellular phone, computer, slate personal computer ("PC"), or a tablet PC (e.g., Apple^{®} iPad^{®}). In some situations, the communications device 340 includes an interface, such as a graphical user interface (GUI), for enabling the user 310 to access the server 315 and the POC system 305. This may enable the user 310 to retrieve dose information or upload concentration data, for example, or monitor the user's 310 treatment. For example, the communications device 340 may enable the user 310 to determine whether the user's drug concentration profile is as desired.

In some examples, the POC instrument includes features described in U.S. Patent No. 7,635,594 ("POINT-OF-CARE FLUIDIC SYSTEMS AND USES THEREOF") or PCT Patent Publication No. WO/2009/046227 ("MODULAR POINT-OF-CARE DEVICES, AND USES THEREOF"). Point of service systems (or devices) provided herein can be as described in U.S. Patent Application No. 13/244,946 to Holmes ("SYSTEMS AND METHODS FOR COLLECTING AND TRANSMITTING ASSAY RESULTS") or U.S. Patent Application No. 13/244,947 to Holmes et al. ("SYSTEMS AND METHODS FOR MULTI-ANALYSIS"). A point of service system can perform one or more, 2 or more, 3 or more, 4 or more, 5 or more, 10 or more, 20 or more, 30 or more, 40 or more, 50 or more, or 100 or more assays on a sample. In some cases, a point of service system can perform sample processing, subsequent data processing and, in some cases, data analysis. A point of service system can perform sample processing and transmit pre-processed data to a remote system for data processing and, in some cases, analysis.

In some situations, a point of service system is configured to process a retrieved sample from a subject and provide data for subsequent analysis. Such analysis can be performed by the point of service system and/or a remote system in communication with the point of service system. In an exemplary implementation, a point of service system can perform centrifugation on a sample to generate data that is subsequently analyzed by the point of service system and/or a remote system.

In some examples, a point of service includes a camera that is adapted to capture a two-dimensional and/or three-dimensional representation of a subject, which can aid in monitoring the progression of a drug treatment. In an example, the camera can collect data that is relevant to one or more physical indicators of the subject (e.g., facial expression, pupil dilation), which can aid in monitoring drug treatment (see below).

In some examples, a point of service system can include a plurality of modules mounted on a support structure. An individual module of the plurality of modules can comprise a sample preparation station, assay station, and/or detection station. In addition, the system can be configured to perform (a) at least one sample preparation procedure selected from the group consisting of sample processing, centrifugation, separation, and chemical processing, and (b) multiple types of assays selected from the group consisting of immunoassay, nucleic acid assay, receptor-based assay, cytometric assay, colorimetric assay, enzymatic assay, electrophoretic assay, electrochemical assay, spectroscopic assay, chromatographic assay, microscopic assay, topographic assay, calorimetric assay, turbidimetric assay, agglutination assay, radioisotope assay, viscometric assay, coagulation assay, clotting time assay, protein synthesis assay, histological assay, culture assay, osmolarity assay, and combinations thereof. The multiple types of assays are performed with the aid of isolated assay units contained within the system.

In some situations, a point of service system can access a network with the aid of systems and methods disclosed in U.S. Patent Application No. 13/244,836 to Balwani ("NETWORK CONNECTIVITY SYSTEMS AND METHODS").

Systems provided herein can be used to renew prescriptions, initiate shipping of the drug or change the prescription to a new drug. In an example, a system monitoring the progression of the health of a subject in response to a particular drug can be configured to recommend and/or prescribe a new drug if the system determines that the subject is not responding to a given drug.

Point of care systems described herein, such as the system 305, can measure the concentration of a drug or metabolite thereof at a rate of one measurement in a time period of no more than 1 week, 72 hours, 48 hours, 24 hours, 12 hours, 8 hours, 4 hours, 2 hours, 60 minutes, 30 minutes, 25 minutes, 20 minutes, 15 minutes, 10 minutes, 5 minutes, 4 minutes, 3 minutes, 2 minutes, 1 minutes, 30 seconds, 10 seconds, 5 seconds, 1 second, 0.5 seconds, or 0.1 seconds. Point of care systems can adjust the dose of a drug at least every 0.1 seconds, 0.5 seconds, 1 second, 10 seconds, 30 seconds, 1 min, 2 min, 3 min, 4 min, 5 min, 6 min, 7 min, 8 min, 9 min, 10 min, 30 min, 1 hour, 2 hours, 3 hours, 4 hours, 5 hours, 6 hours, 7 hours, 8 hours, 9 hours, 10 hours, 11 hours, 12 hours, 1 day, or 2 days.

### Methods for monitoring and regulating the concentration of a drug in a subject

In another example, methods for administering a pharmaceutical composition to a subject are provided. Methods provided herein may be implemented with the aid of systems described above.

In some examples, a method for administering a pharmaceutical composition to a subject (e.g., patient) comprises administering to a subject a first dose of a pharmaceutical composition at a first time point. The first dose of the pharmaceutical composition (or "drug") may be applied to the subject with the aid of a healthcare specialist, such as a doctor, nurse, or other healthcare provider, or a device or apparatus for self-administering the drug. For example, the subject may self-administer the drug with the aid of a syringe having the drug or the subject may ingest the drug.

Next, with the aid of a point of care ("POC") system, a concentration of an active ingredient of the pharmaceutical composition (or a metabolite thereof) or a biological marker indicative of the concentration of the pharmaceutical composition in the subject is determined from a biological sample of the subject, such as a fluid (e.g, blood, saliva) or tissue (e.g., skin) sample. The blood concentration may be determined by measuring the blood concentration of an active ingredient of the pharmaceutical composition or a metabolite thereof. In some implementations, the concentration of an inactive ingredient of the pharmaceutical composition is determined. In some situations, information as to the concentration of an inactive ingredient may be useful in determining the concentration of an active ingredient (or metabolite of the active ingredient) if the concentration of the active ingredient is related to the concentration of the inactive ingredient. This is applicable to the situation where an analyte cannot be measured in the POC system, but another marker may serve as a surrogate for the drug. This method can minimally provide information about drug assimilation.

The POC system may be operatively coupled to the blood or tissue of the subject. For instance, the POC system may include one or more devices for performing an assay to measure the blood concentration of the drug (or metabolite thereof). Blood concentration may be measured in real-time. In some situations, the POC system measures the blood concentration and stores the concentration in a memory or cache of the POC system for subsequent retrieval. Alternatively, the blood concentration may be measured at predetermined intervals, such as at least every 0.1 seconds, 1 second, 2 seconds, 3 seconds, 4 seconds, 5 seconds, 10 seconds, 30 seconds, 1 minute, 30 minutes, 1 hour, 2 hours, 3 hours, 4 hours, 5 hours, 6 hours, 7 hours, 8 hours, 9 hours, 10 hours, 11 hours, 12 hours, or 24 hours. In some situations, the blood concentration is measured at intervals that are calculated or updated by the POC system. For instance, the POC system may determine the best time to take the next biological sample and the type of samples (or analytes) that may be most informative for determining a concentration of the drug or metabolite of the drug in the subject under treatment.

Next, a second dose of the pharmaceutical composition is administered to the subject at a second time point. The second dose and/or the interval between the first and second time points is determined based on the concentration of the pharmaceutical composition in the subject. In some examples, the second dose is administered with the aid of a healthcare provider, such as a doctor (also "physician" herein) or nurse. In other examples, the second dose is administered by the subject under treatment (i.e., self-administered) or with an automatic drug delivery system (e.g., insulin pump). In such a case, the POC system may instruct the subject to self-administer the drug at the second time point at the second dose. In some instances, the second dose is selected based on various features of the subject, such as the subject's physiology (e.g., height and/or weight). For example, subjects with faster metabolisms for a particular drug may require larger doses as compared to subjects with relatively slower metabolisms.

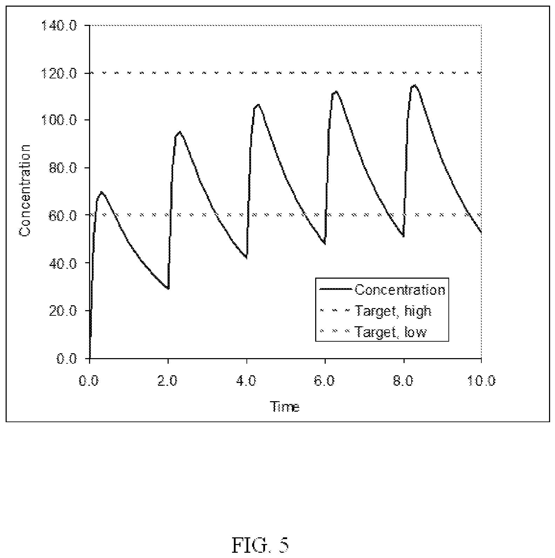

The POC system may compare a measured blood concentration to one or more set-point concentrations and subsequently calculate the second dose based on any differences between the blood concentration and the one or more set-point concentrations. In some situations, the blood concentration is compared to a subject-specific maximum drug concentration and a subject-specific minimum drug concentration. The subject-specific maximum and subject-specific minimum drug concentrations may be determined based on maximum concentrations and minimum concentrations of a plurality of subject-specific drug concentration profiles (i.e., profiles showing blood concentration as a function of time, see FIGs. 5-11).

The POC system may compare the drug concentration to one or more set-point concentrations by comparing the drug concentration to an average set point concentration. The average set-point concentration is a time-averaged set-point concentration. In some situations, the POC system may calculate and store a time-running average of the set-point concentration.

In some implementations, set-point concentrations are subject-specific set-point concentrations. That is, each set-point concentration is calculated by the POC system for a particular subject as opposed to averaging a set-point concentration across a sample group. Subject-specific set-point concentrations may permit the POC system to monitor and regulate the concentration of a drug in a subject on an individual basis, which in some instances is a more accurate approach for treating a subject.

In some implementations, a desirable or predetermined dose is estimated by the POC system before the first dose is administered. In this case, sample measurements are focused on characterizing various subject-specific features, such as a subject's physiology (e.g., height, weight, age, medical history, race, food intake, other drugs taken by the subject, gender, etc.), which may impact drug metabolism and response to the drug. Besides the drug amount, such an approach may be used to select which drug would be most desirable for a subject's treatment.

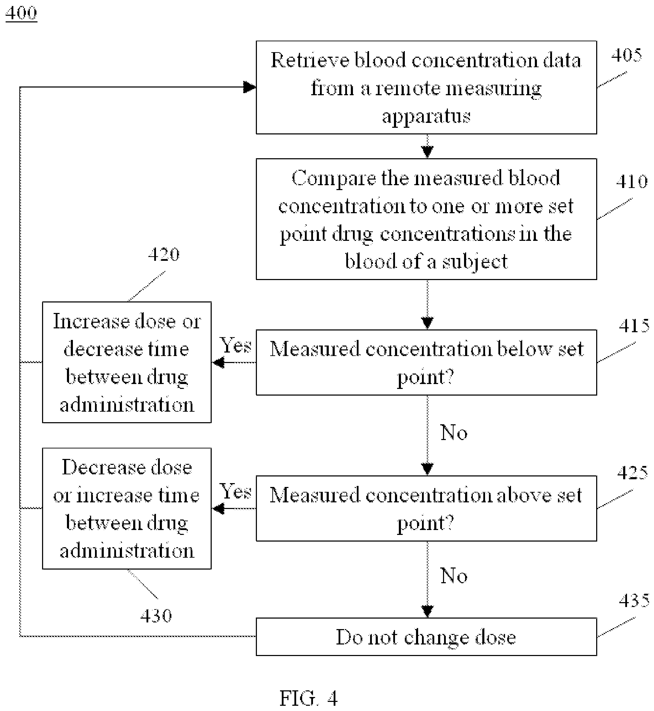

FIG. 4 schematically illustrates a method 400 for monitoring and regulating the concentration of a drug in a subject, in accordance with a method provided herein. The method 400 may be facilitated by a point of care ("POC") system ("the system"), as described above.

In a first step 405, the system retrieves blood concentration data of a drug from a remote measuring apparatus, such as a device for performing an assay on a tissue or fluid sample from a subject. Next, in a second step 410, the measured blood concentration of the drug is compared to one or more set point drug concentrations in the blood of a subject. Next, in a third step 415, the system determines whether the measured blood concentration is below a set point. If the measured blood concentration is below the set point ("Yes"), in step 420 the system may increase the dose or decrease the time between drug administrations, which may increase the effective concentration of the drug (or metabolite of the drug) in the subject. If the measured blood concentration is not below a set point ("No"), in a fourth step 425 the system determines whether the measured blood concentration is above the set point (or, alternatively above another set point which may be different from the set point of step 415). If the measured blood concentration is above the set point, in step 430 the system may decrease the dose or increase the time between drug administrations. Otherwise, if the measured blood concentration is not above the set point, in a fifth step 435 the system does not change the dose. The drug concentration may be dynamic (e.g., after a does is given, it takes time before the maximum drug concentration is reached), so optimal decisions about the next dose may require one or more models that account for the pharmacokinetics and thereby prevent over- and under-shooting around the target drug concentration.

The steps of the method 400 may be repeated to monitor the concentration of the drug at predetermined or calculated intervals, or as requested by a user, such as a subject under treatment or a healthcare provider. For instance, the method 400 may be repeated at least every 0.01 seconds, or 0.1 seconds, or 10 seconds, or 30 seconds, or 1 minute, or 2 minutes, or 3 minutes, or 4 minutes, or 5 minutes, or 6 minutes, or 7 minutes, or 8 minutes, or 9 minutes, or 10 minutes, or 30 minutes, or 1 hour, or 2 hours, or 3 hours, or 4 hours, or 5 hours, 6 hours, or 12 hours, or 1 day, or 2 days, or 3 days, or 4 days, or 5 days, or 6 days, or 1 week, or 2 weeks, or 3 weeks, or 1 month, or 2 months, or 3 months, or 4 months, or 5 months, or 0.5 years, or 1 year.

The system may compare blood concentrations to various set points, which may be static set-points or dynamic set-points. The set-point may be calculated based on the subject's physiology (e.g., height, weight, age, medical history, race, food intake, other drugs taken by the subject, gender, etc.), or provided as an average set-point across a sample population. For instance, the system may use a set-point that has been found to be desirable to a certain percentage of a sample population.

A set-point may remain static (or unchanged) through the course of monitoring the concentration of a drug in a subject. Alternatively, a set-point may be dynamic and updated at certain intervals. For instance, upon monitoring the concentration of a drug (or metabolite of the drug) in a subject a learning engine of the system may monitor and learn of the subject's response to a drug and modify set-points to account for such a response. This may be preferable in instances in which a subject's metabolism is above or below a statistically average metabolism. Dynamic set-point control may thus enable the system to monitor and regulate the concentration of a drug on a subject-specific basis.

In some examples, one or more set-points are calculated or updated with the aid of subject-specific pharmacokinetic data and subject-specific pharmacodynamic data (also "PK-PD data" herein). For example, set-points may be estimated with the aid of approaches described in Rodriguez-Fernandez et al., "A hybrid approach for efficient and robust parameter estimation in biochemical pathways," Biosystems (2006), Feb-Mar, 83(2-3), 248-65; Sheela, "An optimized step-size random search (OSSRS). Computer Methods in Applied Mechanics and Engineering," Volume 19, Issue 1, June 1979, Pages 99-106; Moles et al., "Parameter estimation in biochemical pathways: a comparison of global optimization methods; Genome Res. 2003 Nov;13(11):2467-74; and Gutenkunst et al., "Universally sloppy parameter sensitivities in systems biology models," PLoS Comput Biol. 2007 Oct;3(10): 1871-78.

In some instances, PK-PD data is used to predict a subject's response to a particular drug. The system may implement a PK-PD model for a patient and modify or optimize the model at predetermined intervals or on a continual basis.

Changes in the dynamics of the underlying physiology of individuals may significantly alter the response to on-going drug treatment. For example, liver toxicity may cause a reduction in the drug elimination rate or the introduction of cross-reactivity with administration of another drug. These changes in physiology may compromise the efficacy and safety of the on-going drug treatment; in some instances, the identification of such events followed by corrective action is important in the successful administration of drug therapy. In some examples, drug monitoring may be altered or optimized based on the identification of a change in the behavior of a subject under treatment. For example, once liver toxicity has been identified, the system may alter the dose to account for any increases in the concentration of the drug (or a metabolite of the drug) in a subject under treatment.

As an alternative to the methods provided herein, systems and methods may monitor a drug regimen by measuring and monitoring the concentration of a marker that is indicative of the concentration of the drug in the subject. For example, the concentration of hemoglobin may be measured to monitor and regulate the concentration of a particular drug that has a direct or indirect effect on hemoglobin. In some cases, if a given drug induces a change in hemoglobin levels, such as in the rate of production or loss of hemoglobin, hemoglobin may be used as a surrogate for measuring a concentration of the drug or a metabolite thereof.

In addition to characterizing the pharmacokinetics by measuring the drug or drug surrogates, analytes may be measured that characterize the pharmacodynamics. Such data can further help establish the target drug concentration by balancing the drug level with the effect of the drug (e.g., efficacy and safety). Moreover, the pharmacodynamics provide additional information about the speed of the response to the drug, which may be critical for making optimal decisions about adjusting the dose or selecting the right drug for the subject.

Systems and methods provided herein can be used to monitor the progression of a health condition of a subject, which can enable a particular drug therapy to be tailored to the subject. In some situations, the drug therapy can be tailored in view of various outcomes. For example, the subject's response to a drug can be used to select the dose of the drug and/or the interval at which the drug is administered (or prescribed).

In another example, provided herein are methods for using a point of care system to monitor the treatment and/or progression of a health condition of a subject, and to adjust the concentration of a drug or metabolite thereof in the subject to tailor the treatment and/or progression to a given outcome. In some examples, a point of care system tailors a drug therapy (e.g., drug dose) in order to achieve a desired response by measuring i) biomarkers (e.g., analytes) indicative of the response to assess pharmacokinetics and pharmacodynamics, and/or ii) physical indicators that can be indicative of the subject's response to a prescribed or administered drug. Such an approach advantageously provides subject-specific therapy, as a particular treatment, including drug therapy and associate outcomes, are specific to a subject. In some examples, a drug therapy including the type of drug prescribed and the dose of the drug is selected based on the subject's response to the drug.

In some examples, a subject is treated for a given health condition (e.g., sepsis) by selecting a dose of a drug that can be suited to treat the health condition. In some cases, the initial dose regiment to be given to the subject is selected based on one or more clinical endpoints (e.g., complete response, non-response, partial response, adverse drug reaction) and, in some cases, on established population pharmacokinetic-pharmacodynamic (PK/PD) relationships. Such relationships can help to identify sub-populations of subjects with different dosage requirements, by utilizing demographic data, clinical findings, clinical chemistry results, and/or, when appropriate, pharmacogenetic characteristics.

The concentration of a drug or metabolite thereof can be monitored by the point of care system through a posteriori therapeutic drug monitoring, which can include pre-analytical, analytical and post-analytical phases. The point of care system can determine the active and/or toxic forms of a drug in a biological sample from the subject, which can be collected at given times (or intervals) (pharmacokinetic monitoring). The point of care system in some cases can employ the measurement of a biological perimeter as a surrogate or end-point marker of effect (pharmacodynamic monitoring). For example, the point of care system can measure the concentration of an endogenous compound, enzymatic activity, or gene expression, either as a complement to PK monitoring or as the main therapeutic drug monitoring (TDM) tool. The point of care system can interpret the results, taking into account pre-analytical conditions, clinical information and clinical efficacy of a dosage regimen, such as by applying pharmacokinetic-pharmacodynamic modeling, such as using population PK/PD models and, in some cases, combined with subject-specific pharmacokinetic forecasting techniques, or pharmacokinetic data.

In some examples, a point of care system can be used to monitor a treatment of a subject in response to a given drug therapy. The point of care system can characterize one or more clinical outcomes of a subject, such as, for example, complete response (CR), partial response (PR), stable disease (SR), non-response (NR), adverse drug effect/reaction (ADR), and drug toxicity. Such characterization can made by first constructing a probability space defined by a set of discrete clinical outcomes (e.g., SR, NR, CR, PR, ADR and drug toxicity), each of which is characterized by a statistical distribution of at least one biological marker and/or physical indicator, and then obtaining subject data corresponding to the at least one biological marker and/or at least one physical indicator. The system can then calculate the position of the subject data in the probability space to characterize the probability of a clinical outcome.

In some examples, systems and methods provided herein may be used for the development or implementation of adaptive clinical trials, in order to assess the safety or efficacy of a drug. For example, using systems and methods provided herein, a subject may be administered a first dose of a drug, the subject's response to the drug may be monitored and analyzed, and based on subject's response to the first dose of the drug, a determination regarding a second dose of the drug for the subject may be made (e.g. whether to administer a second dose at all, an appropriate amount of a second dose, timing for a second dose, etc.). Implementation of adaptive clinical trials using systems and methods provided herein may provide numerous advantages during clinical trials for a drug, including, without limitation, improved subject safety (by closely regulating the concentration of a drug in a subject so as to avoid dangerous levels of a drug), better PK and PD data regarding the drug and subject response to the drug, greater subject pool for analysis, and improved data regarding the efficacy of a drug. These advantages may further result in, for example, cheaper and faster clinical trials.

Examples of systems and methods for assessing clinical outcomes are described in U.S. Patent Application No. 12/412,334 to Michelson et al. ("METHODS AND SYSTEMS FOR ASSESSING CLINICAL OUTCOMES") ("Michelson"). The methods of Michelson can be used with systems and methods provided herein to treat a subject receiving a given drug therapy, such as to monitor the progression of a given drug therapy within a probability space having a plurality of discrete clinical outcomes, which can include calculating a trajectory (x(t)) of the progression of the therapy within the probability space, and in some cases calculating a velocity (dx(t)/dt) and/or acceleration (d²x(t)/dt²) from the trajectory. For instance, the rate at which the subject is progressing towards a given outcome (e.g., ADR) in response to a particular drug therapy can be monitored, which can aid the point of care system to make any changes to the therapy that may be necessary to tailor the response to a different outcome (e.g., CR)

### EXAMPLES

### Example 1

FIG. 5 shows a concentration profile for the time course of drug concentration in the blood of a subject when a drug is taken at regular intervals of 2 time units (e.g., hours, days, months). The time and concentration units are arbitrary and provided for illustrative purposes only. The saw tooth pattern is the result of the opposing processes of absorption and distribution of drug into the blood and the elimination and metabolic conversion of the drug to inactive forms. Also shown are the desired upper and lower drug concentrations (or set-points) for therapeutic effectiveness without toxicity.

In this example, the drug dosages are selected conservatively and a therapeutic effect is obtained for about half the time. For some time, the average level of drug increases with each dose of drug. After many doses however, the average drug concentration becomes constant.

### Example 2

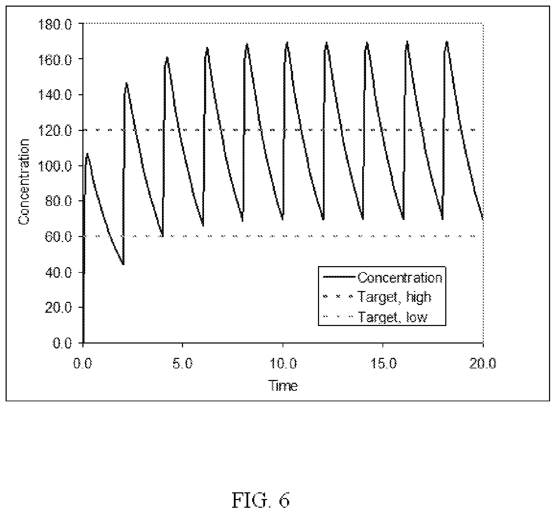

FIG. 6 shows a time course of drug concentration in the blood of a subject. Also shown is a target high set-point and a target low set-point. In the illustrated example, concentration profile maxima are above the target high set-point, indicating that the drug dose is too high, which results in toxicity to the patient throughout the course of treatment. Systems provided herein are used to bring the concentration profile minima and maximum between the target high set point and target low set point.

### Example 3

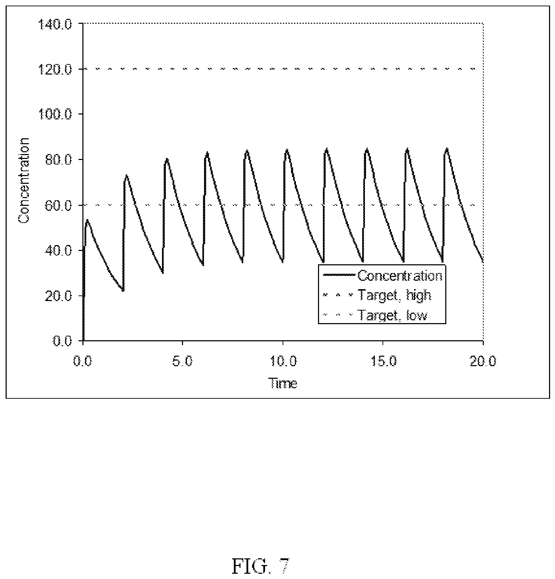

FIG. 7 shows a concentration profile under conditions similar to that of FIG. 6, with the exception that a sub-optimal dose of drug is used, resulting in the therapeutic level not being reached for much of the course of treatment.

### Example 4

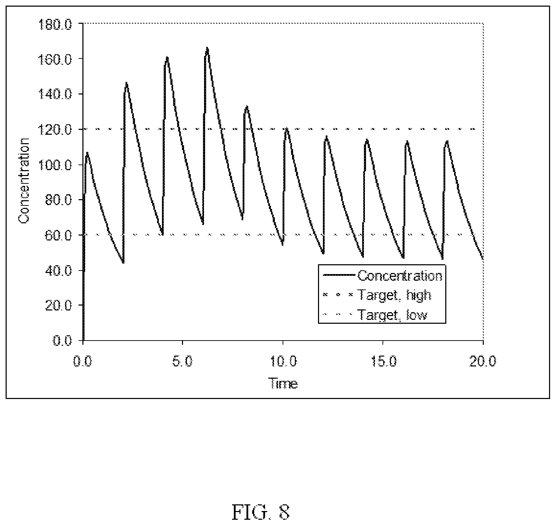

FIG. 8 shows a concentration profile for a case where therapy is being actively managed with the aid of systems and methods described above. After two doses, the drug concentration exceeds the maximum safe level, but by lowering the dosage after four doses (time ~ 8.0), the drug concentration is maintained in the target range where it did not cause appreciable toxicity and is effective for treatment. The use of systems and methods provided herein to make measurements during therapy enables optimal dosage schemes to be implemented.

### Example 5

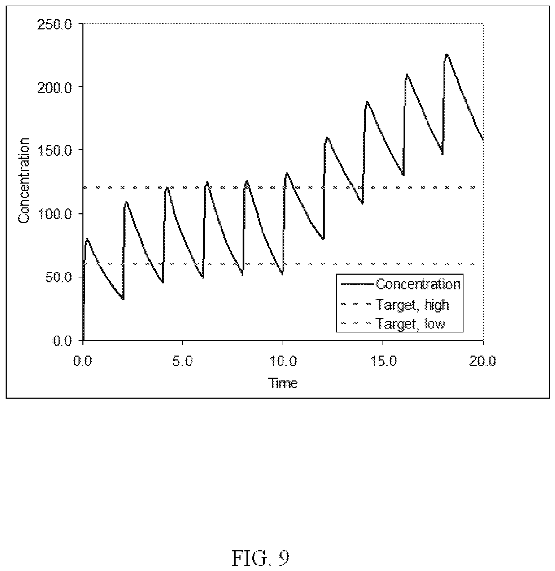

In the illustrated example of FIG. 9, on or after the fifth dose, the elimination rate of the subject drug decreases, potentially due to liver toxicity. Compared to cases in which the drug dose is optimal up to five doses (such as at time 10.0 to 20.0 of FIG. 8), a toxic concentration of the drug is observed. The point of care ("POC") system decreases the dose of the drug to bring the measured drug concentration below the target high set point but above the target low set point.

### Example 6

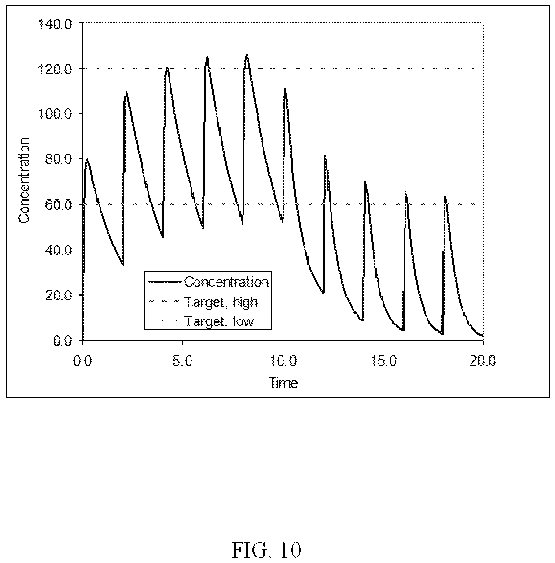

In the illustrated example of FIG. 10, on or after the fifth dose, the administration of another drug induces the production of a higher level of a cytochrome P450 responsible for removal of the first drug, which causes the concentration of the drug to become clinically ineffective. The POC system if used would increase the dose of the drug to bring the drug concentration above the target low set-point and below the target high set-point.

### Example 7

Drug X is used once a month to control the concentration of hemoglobin. In this example, the starting dose is a default value of 0.1 units. Plasma drug concentration and hemoglobin level are sampled by the POC device and analyte data is used in the development of a pharmacokinetic-pharmacodynamic (PK-PD) model. Utilizing the PK-PD model, a dose correction is made at time t = 300 days to achieve a hemoglobin target set-point of 11 g/dL. For comparison, sampling is performed using two alternate sampling designs: (1) fixed sampling design and (2) optimal sampling design (as described herein). In this example, samples of the drug concentration (PK) and hemoglobin (PD) are constrained to occur at the same time (but in the general case can be sampled at different times). The optimal sampling scheme permits management of the drug therapy with fewer data than the fixed scheme, reducing cost and inconvenience to the subject.

Real patient data is obtained by simulating a PK-PD model with the "true" patient parameters. To introduce (simulate) measurement noise, random white noise is added to the "true" simulated data. This approach is geared at least in part to investigating the underlying patient physiologies of interest, as represented by the PK-PD model, by sampling key (or select) biological and molecular analytes. The data that is acquired is then used to infer, or estimate, the model for each patient.

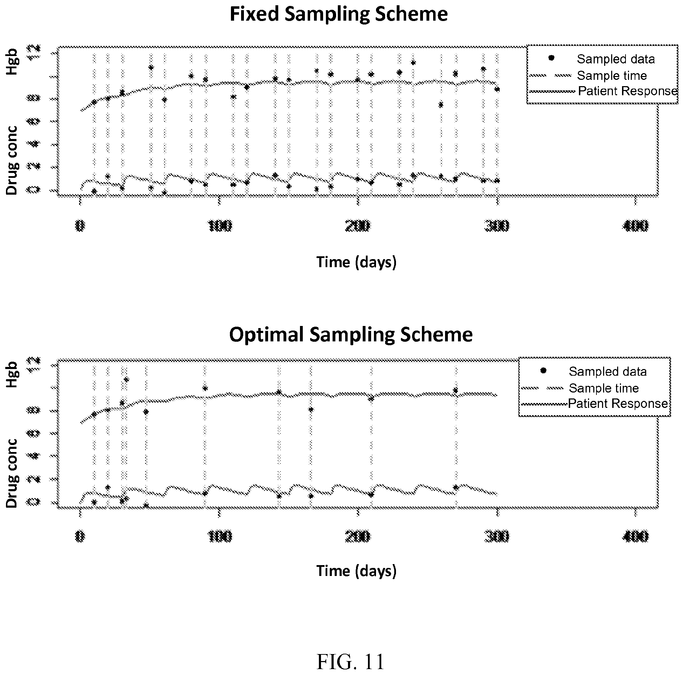

FIG. 11 shows the sampling design for serum drug concentration (mg/L) and hemoglobin (Hgb) (g/dL). The sampling times for the optimal design are determined by the D-optimality criterion to maximize information for the development of a PK-PD model. The fixed sampling design includes twenty-one sampling points and the optimal sampling design includes ten sampling points. The solid black lines represent the actual drug concentration and Hgb levels in a subject over time, while the solid circles represent the simulated assay values that are obtained via a POC assay, subject to noise.

### Example 8

The concentration of hemoglobin in an individual undergoing treatment with drug X is monitored once a month. The dose is fixed to 0.1 units. A PK-PD model is continually updated utilizing all accumulated analyte data of serum drug concentration and hemoglobin concentration obtained by the POC system. The PK-PD model is used to predict the hemoglobin concentration expected at the next sampling time. The prediction error is calculated with each new sample analyzed.

An unexpected increase in the prediction error indicates that a change in patient behavior or physiology has likely occurred (e.g. liver toxicity). The predictions of the PK-PD model are used to detect changes in patient behavior/physiology. Further, using the POC system the sampling frequency is modified to update the PK-PD model to account for the change. For comparison, the PK-PD model is corrected with two sampling designs after the detected change in patient behavior/physiology: (1) a fixed sampling design, as before, and (2) increasing the sampling frequency to once every 4 days over a period of a month followed by the original (fixed) sampling design.

Real patient data is obtained by simulating the PK-PD model with the "true" parameters. To introduce measurement noise, random white noise is added on the "true" simulated data. A change in the "real patient" behavior is introduced by implementing a fourfold increase in the clearance of the drug at 150 days. The serum drug concentration and hemoglobin response of the patient, with and without the change in behavior, in response to the administration of drug X, is shown in FIG. 12A.

FIG. 12B shows the prediction errors for the two sampling designs evaluated when the change in patient behavior occurs. For both sampling designs, the prediction error shows an unexpected sudden increase indicating the occurrence of a behavior change in the subject. The PK-PD model in both cases is corrected using new analyte data after the change in behavior. With the original sampling design, the prediction error is reduced with time; however, the rate of reduction is slow compared to when the POC assay system is flexibly used in the second design to increase the sampling frequency for a period of a month. With this design, the prediction errors are reduced to near optimal performance (errors equivalent to the scenario when no change occurs) within 10 days. The alternate design captures the analyte data at a faster rate and also captures the information of the change in dynamic behavior after the behavior modification has occurred. This advantageously provides analyte data with maximum information content for optimal parameter estimation with the PK-PD model. Such a rapid detection of the change in the subject behavior/physiology, enhanced data sampling, and a learning engine to adjust the dose given the new data, enhances the safety and efficacy for treating the subject.

While the above is a complete description of the preferred examples of the present invention, it is possible to use various alternatives, modifications and equivalents. Therefore, the scope of the present invention should be determined not with reference to the above description but should, instead, be determined with reference to the appended claims. Any feature, whether preferred or not, may be combined with any other feature, whether preferred or not. The appended claims are not to be interpreted as including means-plus-function limitations, unless such a limitation is explicitly recited in a given claim using the phrase "means for." It should be understood that as used in the description herein and throughout the claims that follow, the meaning of "a," "an," and "the" includes plural reference unless the context clearly dictates otherwise. Also, as used in the description herein and throughout the claims that follow, the meaning of "in" includes "in" and "on" unless the context clearly dictates otherwise. Also, as used in the description herein and throughout the claims follow, terms of "include" and "contain" are open ended and do not exclude additional, unrecited elements or method steps. Finally, as used in the description herein and throughout the claims that follow, the meanings of "and" and "or" include both the conjunctive and disjunctive and may be used interchangeably unless the context expressly dictates otherwise. Thus, in contexts where the terms "and" or "or" are used, usage of such conjunctions do not exclude an "and/or" meaning unless the context expressly dictates otherwise.

## Claims

1. A system for recommending a second dosage of a pharmaceutical composition for administration to a subject who has previously received a first dosage of said pharmaceutical composition, comprising means for carrying out the steps of:
(a) obtaining, with the aid of a point of care system (POC) comprising a POC module and a drug-monitoring module, said POC module for monitoring, with the aid of a computer processor, a concentration of an active ingredient of a pharmaceutical composition or a metabolite thereof, or a biological marker indicative of the concentration of said pharmaceutical composition, and the hemoglobin concentration in said subject, and said drug monitoring module for providing one or more set point concentrations of an active ingredient of a pharmaceutical composition or a metabolite thereof and a predicted hemoglobin concentration in the subject who has received a first dose of a pharmaceutical composition at a first time
(b) measuring, with the aid of the point of care system and the biological sample, the concentration of an active ingredient of said pharmaceutical composition or a metabolite thereof, or a biological marker indicative of the concentration of said pharmaceutical composition, and the hemoglobin concentration present in said sample obtained from the subject; and
(c) calculating, with the aid of a computer processor, a recommended second dosage or time interval between dosages based on the pharmaceutical composition concentration measured in step (b) wherein if the measured pharmaceutical composition concentration is below the set point concentration, the system increases dose or decrease time between drug administrations or, if the measured pharmaceutical composition concentration is not below the set point, the system determines whether the measured concentration is above the set point, the system decreases the dose or increases the time between drug administration, or if the measured pharmaceutical composition concentration is not above the set point, the system does not change the dose and
(d) increasing sampling frequency of the sample measurement in step (b) if the measured hemoglobin concentration is different from the predicted hemoglobin concentration,
wherein the biological sample is blood and,
wherein the set point concentration and the predicted hemoglobin concentration is calculated or updated with the aid of subject-specific pharmacokinetic data and subject-specific pharmacodynamic data.

2. The system of Claim 1, further comprising:
comparing, with the aid of a processor, said measured pharmaceutical composition concentration of step (b) to one or more set-point concentrations; and
calculating the second dosage based on the difference between the measured pharmaceutical composition concentration and the one or more set-point concentrations.

3. The system of Claim 2, wherein comparing the pharmaceutical composition concentration measured in step (b) to the one or more set-point concentrations comprises comparing the pharmaceutical composition concentration measured in step (b) to a subject-specific maximum concentration and a subject-specific minimum concentration.

4. The system of Claim 3, wherein subject-specific maximum drug concentration and subject-specific minimum drug concentration are determined based on maximum concentrations and minimum concentrations of a plurality of subject-specific drug concentration profiles.

5. The system of Claim 2, wherein comparing said pharmaceutical composition concentration measured in step (b) to the one or more set-point concentrations comprises comparing the concentration measured in step (b) to an average set point concentration.

6. The system of Claim 5, wherein the average set point concentration is a time-averaged set-point concentration.

7. The system of Claim 1, wherein the point of care system is operatively coupled to the blood or tissue of the subject.

## Patentansprüche

1. System zum Empfehlen einer zweiten Dosierungen einer pharmazeutischen Zusammensetzung für die Verabreichung an ein Subjekt, das zuvor eine erste Dosierung der pharmazeutischen Zusammensetzung erhalten hat, umfassend Mittel zum Ausführen der Schritte:
(a) Erhalten mit der Hilfe eines Point-of-Care-Systems (POC), das ein POC-Modul und ein Arzneimittelüberwachungsmodul umfasst, des POC-Moduls zum Überwachen mit der Hilfe eines Computerprozessors, einer Konzentration eines Inhaltsstoffs einer pharmazeutischen Zusammensetzung oder eines Metaboliten davon oder eines biologischen Markierers, der die Konzentration der pharmazeutischen Zusammensetzung anzeigt, und der Hämoglobinkonzentration in dem Subjekt, und des Arzneimittelüberwachungsmoduls zum Bereitstellen einer oder mehrerer Sollwertkonzentrationen eines Inhaltsstoffs einer pharmazeutischen Zusammensetzung oder eines Metaboliten davon und einer vorhergesagten Hämoglobinkonzentration in dem Subjekt, das eine erste Dosis einer pharmazeutischen Zusammensetzung zu einem ersten Zeitpunkt erhalten hat
(b) Messen mit der Hilfe des Point-of-Care-Systems und der biologischen Probe der Konzentration eines Inhaltsstoffes der pharmazeutischen Zusammensetzung oder eines Metaboliten davon oder eines biologischen Markierers, der die Konzentration der pharmazeutischen Zusammensetzung und die Hämoglobinkonzentration anzeigt, die in der Probe, die von dem Subjekt erhalten wurde, vorhanden ist; und
(c) Berechnen mit der Hilfe eines Computerprozessors einer empfohlenen zweiten Dosierung oder eines Zeitintervalls zwischen Dosierungen auf Grundlage der Konzentration der pharmazeutischen Zusammensetzung, die in Schritt (b) gemessen wurde, wobei, wenn die gemessene pharmazeutische Konzentration unter der Sollwertkonzentration ist, das System die Dosis erhöht oder die Zeit zwischen den Arzneimittelverabreichungen verringert, oder, wenn die gemessene Konzentration der pharmazeutischen Zusammensetzung nicht unter dem Sollwert liegt, das System bestimmt, ob die gemessene Konzentration über dem Sollwert liegt, das System die Dosis verringert oder die Zeit zwischen den Arzneimittelverabreichungen erhöht, oder wenn die gemessene Konzentration der pharmazeutischen Zusammensetzung nicht über dem Sollwert liegt, das System die Dosis nicht ändert, und
(d) Erhöhen der Abtastfrequenz der Probenmessung in Schritt (b), wenn die gemessene Hämoglobinkonzentration sich von der vorhergesagten Hämoglobinkonzentration unterscheidet,
wobei die biologische Probe Blut ist und
wobei die Sollwertkonzentration und die vorhergesagte Hämoglobinkonzentration mit der Hilfe der subjektspezifischen pharmakokinetischen Daten und der subjektspezifischen pharmakodynamischen Daten berechnet oder aktualisiert wird.

2. System nach Anspruch 1, ferner umfassend:
Vergleichen mit der Hilfe eines Prozessors der gemessenen Konzentration der pharmazeutischen Zusammensetzung von Schritt (b) mit einer oder mehreren Sollwertkonzentrationen; und
Berechnen der zweiten Dosierung auf Grundlage des Unterschieds zwischen der gemessenen Konzentration der pharmazeutischen Zusammensetzung und der einen oder den mehreren Sollwertkonzentrationen.

3. System nach Anspruch 2, wobei das Vergleichen der gemessenen Konzentration der pharmazeutischen Zusammensetzung in Schritt (b) mit der einen oder den mehreren Sollwertkonzentration das Vergleichen der gemessenen Konzentration der pharmazeutischen Zusammensetzung in Schritt (b) mit einer subjektspezifischen maximalen Konzentration und einer subjektspezifischen minimalen Konzentration umfasst.

4. System nach Anspruch 3, wobei die subjektspezifische maximale Arzneimittelkonzentration und die subjektspezifische minimale Arzneimittelkonzentration auf Grundlage von maximalen Konzentrationen und minimalen Konzentrationen einer Mehrzahl von subjektspezifischen Arzneimittelkonzentrationsprofilen bestimmt werden.

5. System nach Anspruch 2, wobei das Vergleichen der Konzentration der pharmazeutischen Zusammensetzung in Schritt (b) mit der einen oder den mehreren Sollwertkonzentrationen das Vergleichen der Konzentration, die in Schritt (b) gemessen wurde, mit einer durchschnittlichen Sollwertkonzentration umfasst.

6. System nach Anspruch 5, wobei die durchschnittliche Sollwertkonzentration eine zeitgemittelte Sollwertkonzentration ist.

7. System nach Anspruch 1, wobei das Point-of-Care-System betriebsmäßig mit dem Blut oder dem Gewebe des Subjekts gekoppelt ist.

## Revendications

1. Système de recommandation d'un second dosage d'une composition pharmaceutique pour administration à un sujet ayant préalablement reçu un premier dosage de ladite composition pharmaceutique, comprenant des moyens pour réaliser les étapes suivantes :
(a) l'obtention, à l'aide d'un système de point d'intervention (POC) comprenant un module POC et un module de surveillance de médicament, ledit module POC pour surveiller, à l'aide d'un processeur informatique, une concentration d'un ingrédient actif d'une composition pharmaceutique ou d'un métabolite de celle-ci, ou d'un marqueur biologique indicative de la concentration de ladite composition pharmaceutique et de la concentration d'hémoglobine chez ledit sujet, et ledit module de surveillance de médicament pour fournir une ou plusieurs concentrations de consigne d'un ingrédient actif d'une composition pharmaceutique ou d'un métabolite de celle-ci et une concentration d'hémoglobine prédite chez le sujet qui a reçu une première dose d'une composition pharmaceutique dans un premier temps
(b) la mesure, à l'aide du système de point d'intervention et l'échantillon biologique, de la concentration d'un ingrédient actif de ladite composition pharmaceutique ou d'un métabolite de celle-ci, ou d'un marqueur biologique indicatif de la concentration de ladite composition pharmaceutique, et de la concentration d'hémoglobine présente dans ledit échantillon obtenu à partir du sujet ; et
(c) le calcul, à l'aide d'un processeur informatique, d'une seconde dose recommandée ou d'un intervalle de temps entre les doses sur la base de la concentration de la composition pharmaceutique mesurée à l'étape (b),
dans lequel si la concentration de la composition pharmaceutique mesurée est inférieure à la concentration de consigne,
le système augmente la dose ou diminue le temps entre l'administration du médicament ou,
si la concentration mesurée de la composition pharmaceutique n'est pas inférieure au point de consigne, le système détermine si la concentration mesurée est supérieure au point de consigne, le système diminue la dose ou augmente le temps entre l'administration du médicament, ou
si la concentration de composition pharmaceutique mesurée n'est pas supérieure au point de consigne, le système ne modifie pas la dose et
(d) l'augmentation de la fréquence d'échantillonnage de la mesure d'échantillon à l'étape (b) si la concentration d'hémoglobine mesurée est différente de la concentration d'hémoglobine prédite, dans lequel l'échantillon biologique est du sang et,
dans lequel la concentration de consigne et la concentration d'hémoglobine prédite sont calculées ou mises à jour à l'aide de données pharmacocinétiques spécifiques au sujet et de données pharmacodynamiques spécifiques au sujet.

2. Système selon la revendication 1, comprenant en outre :
la comparaison, à l'aide d'un processeur, de ladite concentration de composition pharmaceutique mesurée de l'étape (b) à une ou plusieurs concentrations de consigne ; et
le calcul du second dosage sur la base de la différence entre la concentration de composition pharmaceutique mesurée et l'une ou plusieurs concentrations de consigne.

3. Système selon la revendication 2, dans lequel la comparaison de la concentration de la composition pharmaceutique mesurée à l'étape (b) à une ou plusieurs concentrations de consigne comprend la comparaison de la concentration de la composition pharmaceutique mesurée à l'étape (b) à une concentration maximale spécifique au sujet et une concentration minimale spécifique au sujet.

4. Système selon la revendication 3, dans lequel la concentration maximale de médicament spécifique au sujet et la concentration minimale de médicament spécifique au sujet sont déterminées sur la base des concentrations maximales et des concentrations minimales d'une pluralité de profils de concentration de médicament spécifique au sujet.

5. Système selon la revendication 2, dans lequel la comparaison de ladite concentration de composition pharmaceutique mesurée à l'étape (b) à une ou plusieurs concentrations de consigne comprend la comparaison de la concentration mesurée à l'étape (b) à une concentration de consigne moyenne.

6. Système selon la revendication 5, dans lequel la concentration de consigne moyenne est une concentration de consigne moyennée dans le temps.

7. Système selon la revendication 1, dans lequel le système de point d'intervention est fonctionnellement couplé au sang ou au tissu du sujet.
